# EUROPÄISCHE PATENTANMELDUNG

(11) **EP 3 785 737 A1**
(43) Veröffentlichungstag der Anmeldung: **03.03.2021**
(21) Anmeldenummer: 20203565.5
(22) Anmeldetag: 13.05.2016
(51) Int. Cl.: A61L 15/24, A61L 15/26, A61L 15/58, B32B 7/12, B32B 27/12, A61F 13/02

(54) **SILIKONGELBESCHICHTETE ADHÄSIVE SCHICHTSTRUKTUR**

(30) Priorität: 18.05.2015 DE 102015107743
(62) Teilanmeldung aus: 16724013.4
(71) Anmelder: BSN medical GmbH, 22761 Hamburg (DE)
(72) Erfinder: Casu, Sascha, 21147 Hamburg (DE); Wogram, Marco Peter, 22113 Oststeinbek (DE); Krause-Kyora, Felix, 22111 Hamburg (DE)
(74) Vertreter: Uexküll & Stolberg

(57) **Zusammenfassung**

Die vorliegende Erfindung betrifft eine silikongelbeschichtete, adhäsive Schichtstruktur, ein Verfahren zu deren Herstellung sowie deren Verwendung. Die Schichtstruktur umfasst ein poröses Trägermaterial, eine auf eine Seite des porösen Trägermaterials aufgetragene Zwischenschicht und eine auf die Zwischenschicht aufgetragene adhäsive Schicht aus einem Silikongel.

## Beschreibung

Die vorliegende Erfindung betrifft eine silikongelbeschichtete, adhäsive Schichtstruktur, ein Verfahren zu deren Herstellung sowie deren Verwendung.

### HINTERGRUND DER ERFINDUNG

Silikonbeschichtete, adhäsive Strukturen zeigen eine Reihe innovativer Eigenschaften, die sie besonders interessant für medizinische Anwendungen machen. So ist ein auf Silikon basierendes Haftmittel (im Folgenden Silikongel genannt) sanfter und angenehmer zur Haut als herkömmliche Acrylat-Klebmassen. Silikongele lassen sich zum Beispiel einfach und unter minimaler Ablösung von Hautzellen und Haaren entfernen und bieten gleichzeitig eine optimale und gleichbleibende Klebkraft über die gesamte Anwendungsdauer. Ein wiederholtes Anwenden ist ebenfalls problemlos möglich. Zudem zeigen Silikongele eine sehr gute Haftung zu anderen in der Struktur vorhandenen Komponenten, zum Beispiel zu einem Acrylsäurecopolymer. Dadurch kann ein Umspulen auf die Haut verringert oder sogar vollständig verhindert werden, wodurch zusätzliche Reinigungsschritte zum Entfernen von Haftmittelrückständen von der Patientenhaut entfallen können. Diese Eigenschaften machen mit Silikongel beschichtete adhäsive Strukturen für die Anwendung in der Medizin, vor allem für eine Anwendung auf empfindlicher und fragiler Patientenhaut, sehr interessant.

Um einen optimalen Tragekomfort zu gewährleisten, sind die Trägermaterialien von in der Medizin verwendeten adhäsiven Strukturen üblicherweise aus einem weichen und elastischen Material gefertigt. Um die unter der adhäsiven Struktur liegende Patientenhaut bestmöglich mit Sauerstoff oder Feuchtigkeit zu versorgen, sind poröse Trägermaterialien besonders vorteilhaft, da diese einen Sauerstoff- und/oder Feuchtigkeitstransport zu der unter der adhäsiven Schichtstruktur liegenden Patientenhaut erlauben. Die Porosität des Trägermaterials führt jedoch oftmals zu Problemen bei der Herstellung von adhäsiven Strukturen auf Silikongelbasis. Silikongele sind vor ihrer Vernetzung fließfähige Massen, die bei einer direkten Beschichtung auf einen porösen Träger in ihn eindringen und oftmals auf der gegenüberliegenden Seite sogar wieder austreten, was als Durchschlagen bezeichnet wird. Dies würde zu einer beschichteten Struktur führen, die auf beiden Seiten eine gewisse Klebrigkeit aufweist, was in der Regel unerwünscht ist.

Silikonbeschichtete, adhäsive Strukturen sind beispielsweise aus der US 2006/0154053 A1 bekannt, in der poröse Träger (Strumpfhosen) offenbart werden, die eine adhäsive Schicht aus einem Silikon oder Acrylat aufweisen. Zwischen dem porösen Träger (dem Strumpf) und der adhäsiven Schicht aus Silikon oder Acrylat befindet sich zudem eine Zwischenschicht aus einem Silikon-Elastomer.

Aus der US 2010/0267302 A1 sind ebenfalls silikonbeschichtete, poröse Träger bekannt.

Ferner sind aus der US 2007/0128263 A1 silikonbeschichtete Substrate bekannt, die eine Zwischenschicht aufweisen. Das Material und die Form der Substrate sind jedoch unbekannt.

### BESCHREIBUNG DER ERFINDUNG

Der vorliegenden Erfindung liegt die Aufgabe zugrunde, eine adhäsive Schichtstruktur zur Verfügung zu stellen, die ein poröses Trägermaterial und eine Haftschicht aus einem Silikongel auf nur einer Seite des porösen Trägermaterials umfasst, wobei ein Eindringen oder Durschlagen des Silikongels in oder durch das poröse Trägermaterial verhindert werden soll. Darüber hinaus soll eine gute Anbindung der adhäsiven Silikongelschicht an die Schichtstruktur gewährleistet sein, um ein unerwünschtes Umspulen zu verringern oder bestenfalls vollständig zu verhindern. Des Weiteren soll ein Verfahren zur Herstellung einer solchen adhäsiven Struktur zur Verfügung gestellt werden.

Diese Aufgaben werden durch eine silikongelbeschichtete, adhäsive Schichtstruktur gelöst, die eine erste Schicht bestehend aus einem porösen Trägermaterial, eine vernetzte oder unvernetzte Zwischenschicht auf nur einer Seite des porösen Trägermaterials und eine Schicht aus einem vernetzten, adhäsiven Silikongel auf der Zwischenschicht umfasst. Weiterhin werden diese Aufgaben durch ein Verfahren zur Herstellung der silikonbeschichteten, adhäsiven Schichtstruktur gelöst, bei dem ein Zwischenschichtmaterial teilweise oder vollständig bedeckend auf ein poröses Trägermaterial aufgetragen wird, das Zwischenschichtmaterial gegebenenfalls vernetzt wird, ein Silikongel auf das vernetzte oder unvernetzte Zwischenschichtmaterial aufgetragen wird und das Silikongel anschließend vernetzt wird. Die Zwischenschicht wirkt dabei als Barriereschicht für das Silikongel und verhindert das Eindringen oder Durchschlagen des Silikongels in oder durch das poröse Trägermaterial, Darüber hinaus bewirkt die Zwischenschicht eine gute Anbindung des Silikongels an die Schichtstruktur.

Die erfindungsgemäße Schichtstruktur ist bevorzugt ein medizinisches Produkt, besonders bevorzugt ein Rollenpflaster, insbesondere ein Rollenpflaster zur Befestigung von medizinischen Instrumenten an der Haut eines Menschen oder Tieres.

Die adhäsive Schichtstruktur, sowie deren Herstellung und Verwendung wird im Folgenden anhand ihrer verschiedenen Ausführungsformen genauer beschrieben.

### Silikonbeschichtete, adhäsive Schichtstruktur

Erfindungsgemäß umfasst die adhäsive Schichtstruktur eine erste Schicht bestehend aus einem porösen Trägermaterial, eine vernetzte oder unvernetzte Zwischenschicht auf einer Seite des porösen Trägermaterials und eine Schicht aus einem vernetzten, adhäsiven Silikongel auf der Zwischenschicht.

### Das poröse Trägermaterial

Um einen bestmöglichen Tragekomfort zu ermöglichen, ist das Trägermaterial erfindungsgemäß aus einem weichen und elastischen Material gefertigt.

Das Trägermaterial kann aus Naturstoff oder Kunststoff hergestellt sein. Hierbei kommen insbesondere Viscose, Rayon, Cellulose, Polyethylen, Polypropylen, Polyamid, Polyester, Polyacetat, Polyurethan oder einer Mischung davon in Frage. Bevorzugt ist das Trägermaterial aus Polyester gefertigt.

Um einen bestmöglichen Sauerstoff- und Feuchtigkeitstransport durch die adhäsive Schichtstruktur zur Haut des Menschen oder Tieres zu gewährleisten, ist das erfindungsgemäße Trägermaterial porös.

Gemäß einer Ausführungsform liegen die oben genannten Materialien in Form eines Vlieses, Gewebes, Gewirkes, Schaumstoffes oder einer Folie vor. Bevorzugt liegen die oben genannten Materialien in Form eines Vlieses vor.

### Zwischenschichtmaterial

Um ein Eindringen oder Durschlagen des adhäsiven Silikongels in oder durch den porösen Träger zu verhindern, ist zwischen dem porösen Träger und der Silikongelschicht eine Zwischenschicht vorhanden. Bevorzugt liegt das Zwischenschichtmaterial auf nur der Silikongel zugewandten Seite des porösen Trägermaterials vor; die andere Seite des porösen Trägermaterials ist frei von Zwischenschichtmaterial. Die Zwischenschicht kann vernetzt oder unvernetzt vorliegen.

Gemäß einer Ausführungsform ist das poröse Trägermaterial vollständig mit der vernetzten oder unvernetzten Zwischenschicht bedeckt.

Gemäß einer weiteren Ausführungsform ist das poröse Trägermaterial teilweise mit der vernetzten oder unvernetzten Zwischenschicht bedeckt. Als teilweise wird im Rahmen dieser Erfindung jede gleichmäßig verteilte Bedeckung des porösen Trägermaterials mit dem Zwischenschichtmaterial verstanden, die nicht vollständig ist.

Die mit dem Zwischenschichtmaterial bedeckte Seite des porösen Trägermaterials ist im Bereich von 30 bis 100%, bevorzugt 40 bis 100%, bevorzugter 50 bis 100%, noch bevorzugter 60 bis 100%, am bevorzugtesten 70 bis 100%, insbesondere 80 bis 100%, mit dem Zwischenschichtmaterial bedeckt.

Die vernetzte oder unvernetzte Zwischenschicht liegt erfindungsgemäß in einer Menge von 10 bis 500 g/m², bevorzugt 10 bis 400 g/m², bevorzugter 10 bis 300 g/m², noch bevorzugter 10 bis 200 g/m², besonders bevorzugt 10 bis 100 g/m², am bevorzugtesten 33 g/m², auf dem porösen Trägermaterial vor.

Das Vorliegen der erfindungsgemäßen Zwischenschicht führt dazu, dass ein Eindringen oder Durchschlagen des adhäsiven Silikongels auch dann verhindert werden kann, wenn das Zwischenschichtmaterial das poröse Trägermaterial nur teilweise bedeckt, da die Fließfähigkeit des Silikongels durch Vernetzung zum Erliegen kommt, bevor das Silikongel in den unbedeckten Bereichen des porösen Trägermaterials zum porösen Trägermaterial fließen kann.

Gemäß einer Ausführungsform umfasst das Zwischenschichtmaterial ein Acrylsäurecopolymer. Gemäß einer bevorzugten Ausführungsform besteht die Zwischenschicht aus dem Acrylsäurecopolymer. Gemäß einer bevorzugteren Ausführungsform ist das Acrylsäurecopolymer aus Monomeren der Gruppe bestehend aus Acrylsäure, Acrylsäurebutylester, 2-Ethylhexylacrylat, 2-Hydroxyethylacrylat, Dioctylacrylat, Methylacrylat, Ethylacrylat, tert-Butylacrylat und Mischungen davon hergestellt.

### Silikongel

Die adhäsive Schichtstruktur umfasst ferner ein auf die vernetzte oder unvernetzte Zwischenschicht aufgetragenes adhäsives Silikongel. Bevorzugt liegen das Zwischenschichtmaterial und das adhäsive Silikongel auf nur einer Seite des porösen Trägermaterials vor; die andere Seite des porösen Trägermaterials ist frei von Zwischenschichtmaterial und adhäsivem Silikongel.

Silikongel bedeutet im Rahmen dieser Erfindung ein aus mindestens einem Silikon-Grundmaterial und einem Katalysator zusammengesetztes Silikonmaterial. Silikon-Grundmaterial bedeutet im Rahmen dieser Erfindung ein Silikonmaterial im unvernetzten oder gering vernetzten Zustand, das als Mischung mit mindestens einem Katalysator ein Silikongel ergibt. Das Silikon-Grundmaterial kann dabei in monomerer oder polymerer Form vorliegen. Der Katalysator dient dazu, das Silikon-Grundmaterial zu vernetzen.

Erfindungsgemäße Silikon-Grundmaterialien sind zum Beispiel vinyl-funktionelle Polydimethylsiloxane oder Wasserstofffunktionelle Siloxane, zum Beispiel Hydro-methyl Siloxane. Der Katalysator ist aus üblichen, dem Fachmann bekannten Katalysatoren, die zur Vernetzung von Silikongelen verwendet werden, ausgewählt. Bevorzugt umfasst der Katalysator ein Edelmetall. Besonders bevorzugt umfasst der Katalysator Platin, Palladium oder Nickel, insbesondere Platin. Insbesondere wird der sogenannte Karstedt-Katalysator verwendet. Bevorzugt werden kommerziell erhältliche Silikonkits verwendet, die ein Silikon-Grundmaterial und einen Katalysator umfassen, wobei das Silikon-Grundmaterial und der Katalysator dabei räumlich getrennt voneinander vorliegen, sodass keine ungewollte Vernetzung einsetzt. Erst kurz vor Auftragen des Silikongels auf die Zwischenschicht werden die Komponenten miteinander gemischt. Erfindungsgemäß verwendete Silikonkits sind zum Beispiel das MED 6342 Silikonkit. der NuSil Technology LLC oder das Silpuran 2110 A/B Silikonkit der Wacker Chemie AG.

Gemäß einer Ausführungsform liegt das adhäsive Silikongel in einer Menge von 10 bis 500 g/m², bevorzugt 50 bis 200 g/m², am bevorzugtesten 80 bis 120 g/m², insbesondere 100 g/m², auf der vernetzten oder unvernetzten Zwischenschicht vor.

Bevorzugt ist die erfindungsgemäße silikonbeschichtete, adhäsive Schichtstruktur ein medizinisches Produkt, wie zum Beispiel ein Rollenpflaster oder ein Tape.

Besonders bevorzugt wird die erfindungsgemäße silikonbeschichtete, adhäsive Schichtstruktur zur Befestigung an der Haut eines Menschen oder Tieres verwendet. Dies kann beispielsweise dazu dienen medizinische Instrumente an der Haut des Menschen oder Tieres zu befestigen.

### Verfahren

Die Erfindung betrifft ferner ein Verfahren zur Herstellung der erfindungsgemäßen adhäsiven Schichtstruktur.

Erfindungsgemäß wird zunächst ein Zwischenschichtmaterial auf ein poröses Trägermaterial aufgetragen. Bevorzugt wird das Zwischenschichtmaterial auf nur eine Seite des porösen Trägermaterials aufgetragen, wobei die andere Seite des porösen Trägermaterials frei von Zwischenschichtmaterial ist.

Gemäß einer Ausführungsform wird das Zwischenschichtmaterial vollständig bedeckend auf das poröse Trägermaterial aufgetragen. Gemäß einer anderen Ausführungsform wird das Zwischenschichtmaterial teilweise bedeckend auf das poröse Trägermaterial aufgetragen. Gegebenenfalls wird das Zwischenschichtmaterial nach dem Auftragen auf das poröse Trägermaterial vernetzt. Danach wird ein Silikongel auf das vernetzte oder unvernetzte Zwischenschichtmaterial aufgetragen. Bevorzugt wird das Silikongel nur auf das Zwischenschichtmaterial aufgetragen und nicht auf die von Zwischenschichtmaterial freie Seite des porösen Trägermaterials, sodass eine Seite des porösen Trägermaterials frei von Zwischenschichtmaterial und Silikongel ist. Schließlich wird das Silikongel vernetzt.

### Auftragen der Zwischenschicht

Gemäß einer Ausführungsform wird das Zwischenschichtmaterial in einer unvernetzten oder vernetzten Form direkt auf das poröse Trägermaterial vollständig oder teilweise bedeckend aufgetragen und danach gegebenenfalls (weiter)vernetzt.

Gemäß einer bevorzugten Ausführungsform wird das Zwischenschichtmaterial nicht sofort auf den porösen Träger aufgetragen, sondern zunächst auf ein Substratmaterial, beispielsweise silikoniertes Trennpapier. Anschließend wird das Zwischenschichtmaterial von diesem Substratmaterial mittels Transferbeschichtung, zum Beispiel Kaschieren, vollständig oder teilweise bedeckend auf den porösen Träger aufgetragen.

Gegebenenfalls wird das Zwischenschichtmaterial nach dem Auftragen auf das poröse Trägermaterial und bevor das Silikongel auf die Zwischenschicht aufgetragen wird, vernetzt.

Gemäß einer Ausführungsform wird das Zwischenschichtmaterial dabei thermisch vernetzt. Dies umfasst erfindungsgemäß jedes Vernetzen, bei dem die nötige Energie durch Wärme (thermisch) bereitgestellt wird. Daher umfasst thermisches Vernetzen im Rahmen dieser Erfindung auch ein Vernetzen bei Umgebungstemperatur, beispielsweise Raumtemperatur. Die Vernetzungsdauer ist dabei reziprok abhängig von der zugeführten Wärmeenergie (also der Temperatur). Je höher die Vernetzungstemperatur ist, desto geringer ist die Vernetzungsdauer und umgekehrt.

Gemäß einer anderen Ausführungsform wird das Zwischenschichtmaterial durch UV-Strahlung vernetzt. Dies umfasst erfindungsgemäß jedes Vernetzen, bei dem die nötige Energie durch UV-Strahlung bereitgestellt wird.

Mischformen aus thermischem- und UV-Vernetzen sind ebenfalls im Rahmen dieser Erfindung. So ist es beispielsweise möglich, dass das Vernetzen bei einer erhöhten Temperatur erfolgt und die Zwischenschicht zusätzlich mit UV-Strahlung bestrahlt wird.

Vorzugsweise wird das Zwischenschichtmaterial durch das poröse Trägermaterial hindurch mittels UV-Strahlung vernetzt. Die UV-Strahlung wird also nicht direkt auf das Zwischenschichtmaterial angewendet, sondern auf das poröse Trägermaterial. Auf diese Weise wird eine besonders gute Anbindung zwischen porösem Trägermaterial und der Zwischenschicht gewährleistet.

Bei einer Transferbeschichtung liegt das Zwischenschichtmaterial vor dem Auftragen auf das Substratmaterial vorzugsweise in einem Lösemittel gelöst, bevorzugt Wasser, Benzin oder Aceton, vor. In diesem Fall wird das in dem Lösemittel gelöste Zwischenschichtmaterial auf das Substratmaterial aufgetragen und das Lösemittel anschließend entfernt, bevorzugt verdampft. Dies kann beispielsweise in einem Umluft-Schwebetrockner bei 180°C erfolgen.

Gemäß einer bevorzugten Ausführungsform wird das auf dem Substratmaterial vorliegende Zwischenschichtmaterial vernetzt bevor es mittels Transferbeschichtung auf das poröse Trägermaterial vollständig oder teilweise bedeckend aufgetragen wird. Das Vernetzen erfolgt thermisch oder mittels UV-Strahlung. Mischformen aus thermischem- und UV-Vernetzen sind ebenfalls im Rahmen dieser Erfindung.

Das Zwischenschichtmaterial wird im Allgemeinen in einer Menge von 10 bis 500 g/m², bevorzugt 10 bis 400 g/m², bevorzugter 10 bis 300 g/m², noch bevorzugter 10 bis 200 g/m², besonders bevorzugt 10 bis 100 g/m², am bevorzugtesten 33 g/m², auf das poröse Trägermaterial aufgetragen. Hierbei werden 30 bis 100%, bevorzugt 40 bis 100%, bevorzugter 50 bis 100%, noch bevorzugter 60 bis 100%, am bevorzugtesten 70 bis 100%, insbesondere 80 bis 100%, einer Seite des porösen Trägermaterials mit dem Zwischenschichtmaterial bedeckt.

### Auftragen der Silikongelschicht auf die vernetzte oder unvernetzte Zwischenschicht

Nachdem die Zwischenschicht auf das poröse Trägermaterial aufgetragen und gegebenenfalls vernetzt wurde, wird ein adhäsives Silikongel auf das vernetzte oder unvernetzte Zwischenschichtmaterial aufgetragen. Das Silikongel kann beispielsweise mit einer Schichtrakel aufgetragen werden. Dabei wird das Silikongel bevorzugt nur auf das Zwischenschichtmaterial aufgetragen und nicht auf die von Zwischenschichtmaterial freie Seite des porösen Trägermaterials, sodass eine Seite des porösen Trägermaterials frei von Zwischenschichtmaterial und Silikongel ist.

Das adhäsive Silikongel wird in einer Menge von 10 bis 500 g/m², bevorzugt 50 bis 200 g/m², am bevorzugtesten 80 bis 120 g/m², insbesondere 100 g/m², auf die vernetzte oder unvernetzte Zwischenschicht aufgetragen.

Das Silikongel wird nach dem Auftragen vernetzt. Gemäß einer bevorzugten Ausführungsform wird das Silikongel thermisch vernetzt. Dies kann beispielsweise dadurch geschehen, dass die Schichtstruktur durch einen Trockenkanal geführt wird. Die genaue Vernetzungstemperatur und -dauer wird der Fachmann in Abhängigkeit von dem jeweiligen Silikongel einstellen.

Bevorzugt ist das Silikongel so ausgestaltet, dass die Schichtstruktur gut auf der Haut eines Patienten haftet, sich einfach und unter minimaler Ablösung von Hautzellen und Haaren von der Haut entfernen lässt, eine optimale und gleichbleibende Klebkraft über die gesamte Anwendungsdauer zeigt und gleichzeitig sanft und angenehm zur Haut ist. Ferner werden die technischen Parameter so gewählt, dass die Schicht aus Silikongel eine sehr gute Verankerung mit der darunter liegenden Zwischenschicht aufweist, sodass ein Umspulen auf die Haut verringert und bestenfalls ganz ausgeschlossen werden kann.

Das Silikongel wird bevorzugt erst kurz vor dem Auftragen auf die Zwischenschicht durch Mischen des Silikon-Grundmaterials mit dem Katalysator gebildet, dann auf die Zwischenschicht in der gewünschten Menge aufgetragen und anschließend vernetzt, bevorzugt thermisch. Das Mischungsverhältnis der Komponenten hängt von dem jeweiligen Silikon-Grundmaterial und Katalysator ab und kann je nach Anwendung individuell eingestellt werden.

### AUSFÜHRUNGSBEISPIELE

### Beispiel 1

Es wird ein elastisches, poröses Polyestervlies (Sontara® der Firma DuPont) als Trägermaterial ausgewählt. Das Trägermaterial wird mit einer druckempfindlichen Klebemasse aus Acrylsäurecopolymer (Duro Tak 1154 der Firma Henkel) mittels Transferbeschichtung beschichtet. Für die Transferbeschichtung wird zunächst ein silkonisiertes Trennpapier (G-liner silicone der Firma Mondi) mit einem Schichtrakel mit 33 g/m² Acrylat-Klebemasse beschichtet. Das beschichtete Trennpapier wird in einem Umluft-Schwebetrockner bei 180°C getrocknet und anschließend mit UV-Strahlung einer Intensität von 3,3 mJ/cm² bestrahlt und somit vernetzt. Die Klebmassenseite wird hierbei direkt bestrahlt. Anschließend wird die Acrylatamasse mittels Transferbeschichtung von dem Trennpapier auf das Polyestervlies aufgetragen. Das so entstandene beschichtete Polyestervlies wird erneut durch eine Strahlenkammer geleitet und mit UV-Licht einer Intensität von 265 mJ/cm² bestrahlt. Hierbei wird die Trägerseite bestrahlt und damit die Klebmasse durch den Träger vernetzt. Danach wird das beschichtete Vlies mit einem 2-Komponenten Silikongel (Silpuran 2110 A/B der Wacker Chemie AG) beschichtet. Hierbei wird das Silikongel auf die bereits vorhandene, vernetzte Acrylatschicht aufgetragen. Das Silikongel wird mit einem Auftragsgewicht von 100 g/m² mit einer Schichtrakel aufgetragen. Das beschichtete Vlies wird bei 150°C mit 5 m/min durch einen Trockenkanal geführt und das Silikongel dadurch vernetzt. Das beschichtete Vlies wird abschließend mit einem silikonisiertem Trennpapier eingedeckt.

### Beispiel 2

Zur Herstellung der adhäsiven Schichtstruktur wird ein elastisches, poröses Polyestervlies (Sontara® der Firma DuPont) als Trägermaterial ausgewählt. Das Trägermaterial wird mit einer druckempfindlichen Klebemasse aus Acrylsäurecopolymer (acResin A 260 UV der BASF AG) direkt beschichtet. Bei der Klebemasse handelt es sich um einen Schmelzklebstoff. Dieser wird zunächst bei einer Temperatur von 130°C in einem Fassschmelzer verflüssigt und mit Hilfe einer Schlitzdüse mit einer Auftragsmenge von 33 g/m² direkt auf das Polyestervlies aufgetragen. Auf eine anschließende Trocknung wurde verzichtet. Das beschichtete Vlies wird wie in Beispiel 1 beschrieben mit UV-Licht bestrahlt um die Klebemasse zu vernetzen. Danach wird das beschichtete Vlies mit einem 2-Komponenten Silikongel (Silpuran 2110 A/B der Wacker Chemie AG) beschichtet. Hierbei. wird das Silikongel auf die bereits vorhandene Acrylatschicht aufgetragen. Das Silikongel wird mit einem Auftragsgewicht von 100g/m² mit einer Schichtrakel aufgetragen. Das beschichtete Vlies wird bei 150°C mit 5 m/min durch einen Trockenkanal geführt und das Silikongel dadurch vernetzt. Das beschichtete Vlies wird abschließend mit einem silikonisiertem Trennpapier eingedeckt.

## Patentansprüche

1. Silikongelbeschichtete, adhäsive Schichtstruktur, umfassend:
a) eine erste Schicht bestehend aus einem porösen Trägermaterial,
b) auf einer Seite des porösen Trägermaterials eine vernetzte oder unvernetzte Zwischenschicht und
c) auf der Zwischenschicht eine Schicht aus einem vernetzten, adhäsiven Silikongel.

2. Adhäsive Schichtstruktur nach Anspruch 1, **dadurch gekennzeichnet, dass** die Zwischenschicht und das adhäsive Silikongel nur auf einer Seite des porösen Trägermaterials vorliegt.

3. Adhäsive Schichtstruktur nach einem der vorhergehenden Ansprüche, **dadurch gekennzeichnet, dass** das poröse Trägermaterial ausgewählt ist aus der Gruppe bestehend aus Vliesen, Geweben, Gewirken, Schaumstoffen und Folien, wobei das poröse Trägermaterial vorzugsweise aus Naturstoff oder Kunststoff hergestellt ist, insbesondere Viscose, Rayon, Cellulose, Polyethylen, Polypropylen, Polyamid, Polyester, Polyacetat, Polyurethan oder eine Mischungen davon ist.

4. Adhäsive Schichtstruktur nach einem der vorhergehenden Ansprüche, **dadurch gekennzeichnet, dass** die vernetzte oder unvernetzte Zwischenschicht in einer Menge von 10 bis 500 g/m², bevorzugt von 10 bis 400 g/m², bevorzugter 10 bis 300 g/m², noch bevorzugter 10 bis 200 g/m², besonders bevorzugt 10 bis 100 g/m², am bevorzugtesten 33 g/m², auf dem porösen Trägermaterial vorliegt.

5. Adhäsive Schichtstruktur nach einem der vorhergehenden Ansprüche, **dadurch gekennzeichnet, dass** die vernetzte oder unvernetzte Zwischenschicht Acrylsäurecopolymer umfasst oder daraus besteht, wobei das Acrylsäurecopolymer vorzugsweise aus Monomeren der Gruppe bestehend aus Acrylsäure, Acrylsäurebutylester, 2-Ethylhexylacrylat, 2-Hydroxyethylacrylat, Dioctylacrylat, Methylacrylat, Ethylacrylat, tert-Butylacrylat und Mischungen davon hergestellt ist.

6. Adhäsive Schichtstruktur nach einem der vorhergehenden Ansprüche, **dadurch gekennzeichnet, dass** eine Seite des porösen Trägermaterials von 30 bis 100%, bevorzugt 40 bis 100%, bevorzugter 50 bis 100%, noch bevorzugter 60 bis 100%, am bevorzugtesten 70 bis 100%, insbesondere 80 bis 100%, mit dem Zwischenschichtmaterial bedeckt ist.

7. Adhäsive Schichtstruktur nach einem der vorhergehenden Ansprüche, **dadurch gekennzeichnet, dass** die vernetzte, adhäsive Silikongelschicht in einer Menge von 10 bis 500 g/m², bevorzugt von 50 bis 200 g/m², am bevorzugtesten 80 bis 120 g/m², insbesondere 100 g/m², auf der vernetzten oder unvernetzten Zwischenschicht vorliegt.

8. Adhäsive Schichtstruktur nach einem der vorhergehenden Ansprüche, **dadurch gekennzeichnet, dass** die adhäsive Schichtstruktur ein medizinisches Produkt, bevorzugt ein Rollenpflaster oder ein Tape ist.

9. Verfahren zur Herstellung einer silikongelbeschichteten, adhäsiven Schichtstruktur gemäß einem der vorherigen Ansprüche, bei dem
a) ein Zwischenschichtmaterial teilweise oder vollständig bedeckend auf ein poröses Trägermaterial aufgetragen wird,
b) das Zwischenschichtmaterial gegebenenfalls vernetzt wird,
c) ein Silikongel auf das vernetzte oder unvernetzte Zwischenschichtmaterial aufgetragen wird und
d) das Silikongel vernetzt wird.

10. Verfahren zur Herstellung einer silikongelbeschichteten, adhäsiven Schichtstruktur nach Anspruch 9, bei dem das Zwischenschichtmaterial in Schritt a) nur auf eine Seite des porösen Trägermaterials aufgetragen wird und das Silikongel in Schritt C) nur auf das Zwischenschichtmaterial aufgetragen wird, sodass eine Seite des porösen Trägermaterials frei von Zwischenschichtmaterial und Silikongel ist.

11. Verfahren zur Herstellung einer silikongelbeschichteten, adhäsiven Schichtstruktur nach Anspruch 9 oder 10, bei dem das Zwischenschichtmaterial in Schritt a) dadurch auf das poröse Trägermaterial aufgetragen wird, dass es zunächst auf ein Substratmaterial aufgetragen wird und anschließend von diesem Substratmaterial mittels Transferbeschichtung auf das poröse Trägermaterial aufgetragen wird.

12. Verfahren zur Herstellung einer silikongelbeschichteten, adhäsiven Schichtstruktur nach Anspruch 11, bei dem das Zwischenschichtmaterial
a1) vor dem Auftragen auf das Substratmaterial in einem Lösemittel, bevorzugt Wasser, Benzin oder Aceton, vorliegt,
a2) das Zwischenschichtmaterial auf das Substratmaterial aufgetragen wird und
a3) das Lösemittel anschließend vollständig oder teilweise verdampft wird.

13. Verfahren zur Herstellung einer silikongelbeschichteten, adhäsiven Schichtstruktur nach Anspruch 12, bei dem das auf dem Substratmaterial vorliegende Zwischenschichtmaterial nach Schritt a3) und vor dem Auftragen auf das poröse Trägermaterial vernetzt wird.

14. Verfahren nach einem der Ansprüche 9 bis 13, bei dem das poröse Trägermaterial ausgewählt ist aus der Gruppe bestehend aus Vliesen, Geweben, Gewirken, Schaumstoffen und Folien, wobei das poröse Trägermaterial vorzugsweise aus Naturstoff oder Kunststoff hergestellt ist, insbesondere Viscose, Rayon, Cellulose, Polyethylen, Polypropylen, Polyamid, Polyester, Polyacetat, Polyurethan oder eine Mischung davon ist.

15. Verfahren nach einem der Ansprüche 9 bis 14, bei dem das Zwischenschichtmaterial Acrylsäurecopolymer umfasst oder daraus besteht, wobei das Acrylsäurecopolymer vorzugsweise aus Monomeren der Gruppe bestehend aus Acrylsäure, Acrylsäurebutylester, 2-Ethylhexylacrylat, 2-Hydroxyethylacrylat, Dioctylacrylat, Methylacrylat, Ethylacrylat, tert-Butylacrylat und Mischungen davon hergestellt ist.

16. Verfahren nach einem der Ansprüche 9 bis 15, bei dem das Zwischenschichtmaterial in einer Menge von 10 bis 500 g/m², bevorzugt von 10 bis 400 g/m², bevorzugter 10 bis 300 g/m², noch bevorzugter 10 bis 200 g/m², besonders bevorzugt 10 bis 100 g/m², am bevorzugtesten 33 g/m² auf das poröse Trägermaterial aufgetragen wird.

17. Verfahren nach einem der Ansprüche 9 bis 16, bei dem die vernetzte, adhäsive Silikongelschicht in einer Menge von 10 bis 500 g/m², bevorzugt von 50 bis 200 g/m², bevorzugter 80 bis 120 g/m², insbesondere 100 g/m² auf die vernetzte oder unvernetzte Zwischenschicht aufgetragen wird.

18. Verfahren nach einem der Ansprüche 9 bis 17, bei dem 30 bis 100%, bevorzugt 40 bis 100%, bevorzugter 50 bis 100%, noch bevorzugter 60 bis 100%, am bevorzugtesten 70 bis 100%, insbesondere 80 bis 100%, einer Seite des porösen Trägermaterials mit dem Zwischenschichtmaterial bedeckt werden.

19. Verwendung der silikongelbeschichteten, adhäsiven Schichtstruktur gemäß einem der Ansprüche 1 bis 8 als Medizinprodukt, bevorzugt zur Befestigung an der Haut eines Menschen oder eines Tieres, besonders bevorzugt zur Befestigung von medizinischen Instrumenten an der Haut eines Menschen oder eines Tieres.
